# EUROPEAN PATENT APPLICATION

(11) **EP 4 700 274 A1**
(43) Date of publication of application: **25.02.2026**
(21) Application number: 25197541.3
(22) Date of filing: 22.08.2025
(51) Int. Cl.: F21L 4/00, F21V 5/00, F21V 5/04, F21V 21/084, F21W 131/20, F21Y 115/10

(54) **HEADLIGHT AND HEADLIGHT ASSEMBLY**

(30) Priority: 22.08.2024 US 202463685846 P
(71) Applicant: Metrex Research LLC, Brea, CA 92821 (US)
(72) Inventor: Fritz, John Michael, Verona, 53593 (US); Oren, Matthew Christopher, Brookfield, 53045 (US)
(74) Representative: Cleveland Scott York

(57) **Abstract**

A headlight (12) of the type worn by medical and dental professionals includes a housing (20) having an opening therein. A housing (20) defines opposing ends and a passage. A light source (32), such as a LED, is proximate the first end. An optical assembly (30) is in the passage of the housing (20) and includes a first singlet lens (60), a second singlet lens (62) disposed between the light source (32) and the first singlet lens (60), and a third singlet lens (64) disposed between the light source (32) and the second singlet lens (62). Each of the second singlet lens (62) and the third singlet lens (64) are centered on the optical axis (38) and the second singlet lens (62) and the third singlet lens (64) are spaced apart by a separation distance.

## Description

### TECHNICAL FIELD

The invention relates generally to lighting devices and, more particularly, to portable lighting devices, such as surgical headlights.

### BACKGROUND

Portable lighting devices, such as lighting devices that are to be worn, are well known in the art. In many cases, a user may place a light on their head proximate their face. The light is then directed toward objects in the immediate vicinity of the user. As an example, these types of headlights may be used in surgical procedures. Medical and dental professionals often use surgical headlights to provide additional illumination of a surgical site on a patient. Surgical headlights may be attached to glasses or loupes to be worn by the clinician.

Existing headlights can be deficient in one or more aspects. For one, they may exhibit poor light uniformity and/or unacceptable color separation at the edge of the light beam. In addition, surgical headlights may be too heavy or awkward and are therefore uncomfortable. As such, clinicians consider size, weight, beam quality, light intensity, and ease of adjustment when selecting a headlight for use in surgical procedures.

As an example, to address some of these considerations, a more uniform light beam may be achieved by positioning a singlet lens between a light-emitting diode (LED) and a doublet lens. This arrangement however has a drawback and does not address many of the clinicians' other selection criteria. One drawback, or example, is that the positioning of the doublet lens at the distal opening of the housing results in the doublet lens having a relatively large diameter. Because the doublet lens is relatively heavy, during lengthy surgical procedures, the clinician may experience head and neck discomfort and/or pain.

Therefore, a need exists for a headlight which provides a more uniform light beam, greater light intensity, and which is both lightweight and comfortable.

### SUMMARY

According to a first aspect of the present invention there is provided a headlight of a type worn by medical and dental professionals, the headlight comprising:
a housing defining a first end opposite a second end and a passage extending between the first end and the second end; a light source proximate the first end; and an optical assembly at least partly in the passage of the housing, the optical assembly comprising: a first singlet lens proximate the second end and defining an optical axis; a second singlet lens disposed between the light source and the first singlet lens; and a third singlet lens disposed between the light source and the second singlet lens, wherein each of the second singlet lens and the third singlet lens are centered on the optical axis and the second singlet lens and the third singlet lens are spaced apart by a separation distance.

Preferably the third singlet lens is a plano-convex lens and is oriented with a flat optical surface facing the light source.

Preferably the first singlet lens and the second singlet lens are each bi-convex lenses.

Preferably the optical assembly consists of: the first singlet lens, the second singlet lens, and the third singlet lens.

Preferably the headlight further comprises: a centering ring in the passage proximate the first end and in contact with the third singlet lens and positioning the third singlet lens on the optical axis.

Preferably the centering ring includes a first stop and a second stop, the first stop contacting the second singlet lens and the second stop contacting the third singlet lens, the separation distance being defined by a first distance from the first stop to the second stop.

Preferably the headlight further comprises: a retaining ring in the passage proximate the first end and in contact with the third singlet lens, the retaining ring capturing the third singlet lens against the second stop.

Preferably the headlight further comprises: a printed circuit board on which the light source is mounted, wherein the printed circuit board is at the first end with the light source in the passage and the light source is spaced apart from the third singlet lens by a second distance.

Preferably the housing includes a third stop in the passage, and wherein the first stop of the centering ring holds the second singlet lens against the third stop.

Preferably a third distance from the third stop to the first end is in a range of 4 mm to 6 mm.

Preferably the housing includes a fourth stop proximate the second end, the first singlet lens being in contact with the fourth stop.

Preferably a fourth distance from the third stop to the fourth stop is in a range of 16 mm to 18 mm.

Preferably the light source produces a luminous flux of from 40 lumens to 130 lumens.

Preferably the light source produces a color temperature of from 6300 K to 6700 K.

Preferably the housing defines a longitudinal axis and the optical axis coincides with the longitudinal axis.

According to a second aspect of the present invention there is provided a headlight of the type worn by medical and dental professionals, the headlight comprising:
a housing defining a first end opposite a second end and a passage extending between the first end and the second end;
an LED proximate the first end; and

an optical assembly at least partly in the passage of the housing, the optical assembly consisting of:
   a first singlet lens proximate the second end and defining an optical axis;
   a second singlet lens disposed between the LED and the first singlet lens; and
   a third singlet lens disposed between the LED and the second singlet lens,
wherein each of the second singlet lens and the third singlet lens are centered on the optical axis.

Preferably the third singlet lens is a plano-convex lens and is oriented with a flat optical surface facing the LED.

Preferably the first singlet lens and the second singlet lens are each bi-convex lenses.

Preferably the headlight further comprises: a centering ring in the passage proximate the first end and in contact with the third singlet lens and positioning the third singlet lens on the optical axis.

Preferably the centering ring includes a first stop and a second stop, the first stop contacting the second singlet lens and the second stop contacting the third singlet lens, the separation distance being defined by a first distance from the first stop to the second stop.

Preferably the headlight further comprises: a retaining ring in the passage proximate the first end and in contact with the third singlet lens, the retaining ring capturing the third singlet lens against the second stop.

Preferably the headlight further comprises: a printed circuit board on which the light source is mounted, wherein the printed circuit board is at the first end with the LED in the passage and the light source is spaced apart from the third singlet lens by a second distance.

Preferably the housing includes a third stop in the passage, and wherein the first stop of the centering ring holds the second singlet lens against the third stop.

Preferably a third distance from the third stop to the first end is in a range of 4 mm to 6 mm.

Preferably the housing includes a fourth stop proximate the second end, the first singlet lens being in contact with the fourth stop.

Preferably a fourth distance from the third stop to the fourth stop is in a range of 16 mm to 18 mm.

Preferably the LED produces a luminous flux of from 40 lumens to 130 lumens.

Preferably the LED produces a color temperature of from 6300 K to 6700 K.

Preferably the housing defines a longitudinal axis and the optical axis coincides with the longitudinal axis.

According to a third aspect of the present invention there is provided a headlight of the type worn by medical and dental professionals, the headlight comprising:
a housing defining a first end opposite a second end and a passage extending between the first end and the second end;
a light source proximate the first end; and
an optical assembly at least partly in the passage of the housing, the optical assembly comprising:
   a first bi-convex lens proximate the second end and defining an optical axis;
   a second bi-convex lens disposed between the light source and the first singlet lens; and
   a plano-convex lens disposed between the light source and the second bi-convex lens and the plano-convex lens is oriented with a flat optical surface facing the light source,
wherein each of the second bi-convex lens and the plano-convex lens are centered on the optical axis.

Preferably the optical assembly consists of: the first bi-convex lens, the second bi-convex lens, and the plano-convex lens.

Preferably the headlight further comprises: a centering ring in the passage proximate the first end and in contact with the plano-convex lens and positioning the plano-convex lens on the optical axis.

Preferably the centering ring includes a first stop and a second stop, the first stop contacting the second bi-convex lens and the second stop contacting the plano-convex lens, the separation distance being defined by a first distance from the first stop to the second stop.

Preferably the headlight further comprises: a retaining ring in the passage proximate the first end and in contact with the plano-convex lens, the retaining ring capturing the plano-convex lens against the second stop.

Preferably the headlight further comprises: a printed circuit board on which the light source is mounted, wherein the printed circuit board is at the first end with the light source in the passage and the light source is spaced apart from the plano-convex lens by a second distance.

Preferably the housing includes a third stop in the passage, and wherein the first stop of the centering ring holds the second bi-convex lens against the third stop.

Preferably a third distance from the third stop to the first end is in a range of 4 mm to 6 mm.

Preferably the housing includes a fourth stop proximate the second end, the first bo-convex lens being in contact with the fourth stop.

Preferably a fourth distance from the third stop to the fourth stop is in a range of 16 mm to 18 mm.

Preferably the light source produces a luminous flux of from 40 lumens to 130 lumens.

Preferably the light source produces a color temperature of from 6300 K to 6700 K.

Preferably the housing defines a longitudinal axis and the optical axis coincides with the longitudinal axis.

To these and other ends, embodiments of the invention are directed to a headlight of the type worn by medical and dental professionals. In an exemplary embodiment, the headlight includes a housing that defines a first end opposite a second end. A passage extends between the first end and the second end. A light source is proximate the first end. An optical assembly is at least partly in the passage of the housing. The optical assembly includes a first singlet lens proximate the second end and defines an optical axis. A second singlet lens is disposed between the light source and the first singlet lens. A third singlet lens is disposed between the light source and the second singlet lens. Each of the second singlet lens and the third singlet lens are centered on the optical axis, and the second singlet lens and the third singlet lens are spaced apart by a separation distance.

In one embodiment, the third singlet lens is a plano-convex lens and is oriented with a flat optical surface facing the light source.

In one embodiment, the first singlet lens and the second singlet lens are each bi-convex lenses.

In one embodiment, the first singlet lens is spaced apart from the second singlet lens in a range from 15 mm to 18 mm.

In one embodiment, the second singlet lens is spaced apart from the third singlet lens in a range from 0.2 mm to 0.4 mm.

In one embodiment, the third singlet lens is spaced apart from the light source in a range of from 0.2 mm to 0.4 mm.

In one embodiment, the optical assembly consists of: the first singlet lens, the second singlet lens, and the third singlet lens.

In one embodiment, the light source is a LED having an effective LED die size in a range of 0.3 mm² to 2 mm².

In one embodiment, the headlight further includes a centering ring in the passage proximate the first end and in contact with the third singlet lens and positioning the third singlet lens on the optical axis. In one embodiment, the centering ring includes a first stop and a second stop. The first stop contacts the second singlet lens, and the second stop contacts the third singlet lens. The separation distance is defined by a first distance from the first stop to the second stop. As one example, the first distance is in a range from 1 mm to 2.5 mm.

In one embodiment, the headlight further includes a retaining ring in the passage proximate the first end and in contact with the third singlet lens. The retaining ring captures the third singlet lens against the second stop.

In one embodiment, the headlight further includes a printed circuit board on which the light source is mounted. The printed circuit board is at the first end with the light source in the passage. The light source is spaced apart from the third singlet lens by a second distance. As an example, the second distance between the light source and the third singlet lens is in a range of from 0.2 mm to 0.4 mm.

In one embodiment, the housing includes a third stop in the passage. The first stop of the centering ring holds the second singlet lens against the third stop. As an example, a third distance from the third stop to the first end is in a range of 4 mm to 6 mm.

In one embodiment, the housing includes a fourth stop proximate the second end. The first singlet lens is in contact with the fourth stop. As an example, a fourth distance from the third stop to the fourth stop is in a range of 16 mm to 18 mm.

In one embodiment, the light source produces a luminous flux of from 40 lumens to 130 lumens.

In one embodiment, the light source produces a color temperature of from 6300 K to 6700 K.

In one embodiment, the housing defines a longitudinal axis and the optical axis coincides with the longitudinal axis.

In one embodiment, there is a headlight of the type worn by medical and dental professionals. The headlight includes a housing defining a first end opposite a second end. A passage extends between the first end and the second end. There is an LED proximate the first end. An optical assembly is at least partly in the passage of the housing. The optical assembly consisting of a first singlet lens proximate the second end and defining an optical axis; a second singlet lens disposed between the light source and the first singlet lens; and a third singlet lens disposed between the light source and the second singlet lens. Each of the second singlet lens and the third singlet lens are centered on the optical axis.

In one embodiment, there is a headlight of the type worn by medical and dental professionals. The headlight includes a housing defining a first end opposite a second end. A passage extends between the first end and the second end. There is a light source proximate the first end. An optical assembly is at least partly in the passage of the housing. The optical assembly includes a first bi-convex lens proximate the second end and defining an optical axis; a second bi-convex lens disposed between the light source and the first singlet lens; and a plano-convex lens disposed between the light source and the second bi-convex lens and the plano-convex lens is oriented with a flat optical surface facing the light source. Each of the second bi-convex lens and the plano-convex lens are centered on the optical axis.

### BRIEF DESCRIPTION OF THE DRAWINGS

The accompanying drawings, which are incorporated in and constitute a part of this specification, illustrate embodiments of the invention and, together with the detailed description given below, serve to explain the invention.
Fig. 1 is a perspective view of a headlight assembly in accordance with one embodiment of the present invention; and
Fig. 2 is a cross-sectional view of a headlight shown in Fig. 1, taken along line 2-2.
Fig. 3 is a cross-sectional view of a centering ring in accordance with one embodiment of the invention.

### DETAILED DESCRIPTION

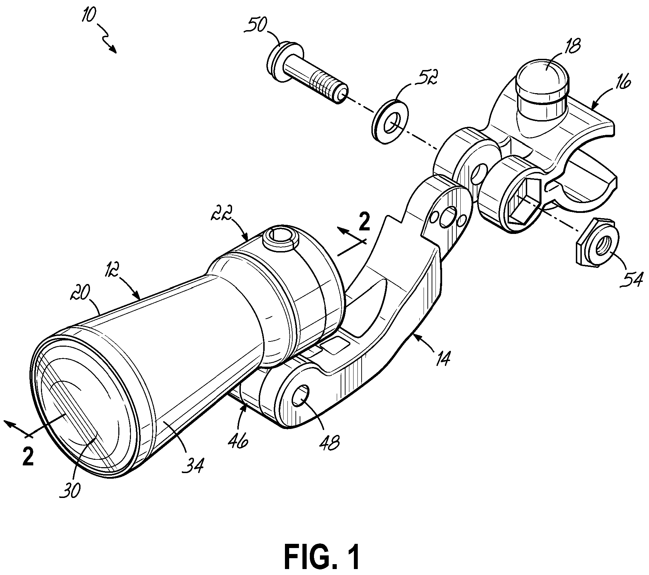

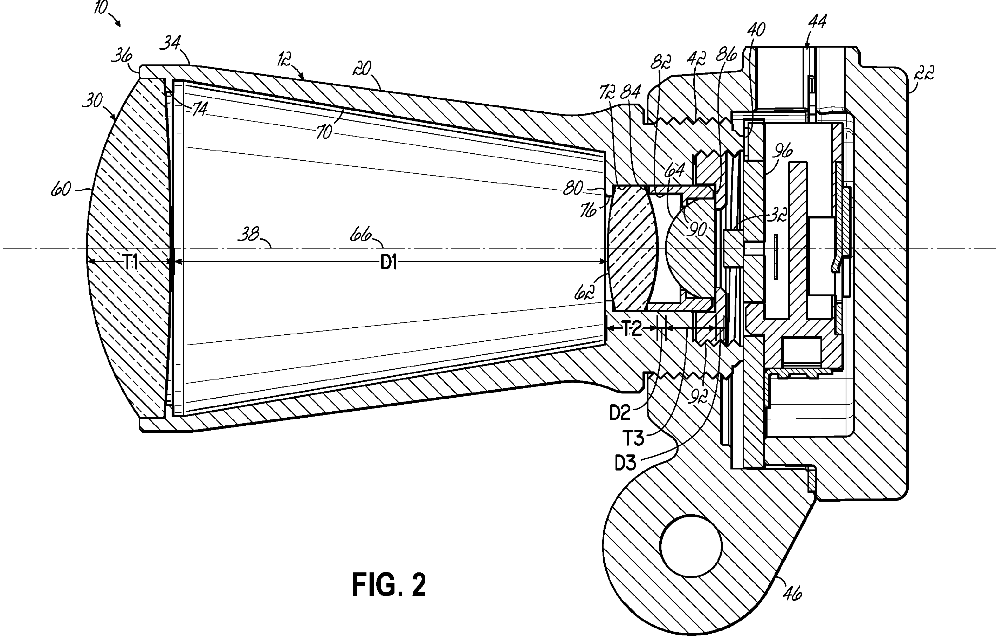

With reference to Figs. 1 and 2, an exemplary embodiment of a headlight assembly 10 includes a headlight having an optics assembly with a series of lenses and a light source that collectively provides an improved light beam having one or more of a greater brightness, greater sharpness, well-defined shape, and greater depth than that disclosed in commonly-owned U.S. Patent No. 10,107,483, which is incorporated herein by reference in its entirety. The headlight assembly 10 may be usable with any loupe, eyewear frame, or headband. To those and other ends and as an example only, the headlight assembly 10 is described with reference to attachment to glasses. However, embodiments of the invention are not limited to that specific point of attachment. The exemplary headlight assembly 10 includes a headlight 12, a yoke 14, and a through-the-lens (TTL) clip 16. The TTL clip 16 may be configured for attachment to a user's glasses or loupes (not shown). For example, the TTL clip 16 may be positioned around a portion of a pair of glasses, and a thumb screw 18 may be tightened to secure the assembly 10 to the glasses.

With reference to Figs. 1 and 2, in the embodiment shown, the headlight 12 includes an optics housing 20 coupled to an electronics housing 22. The optics housing 20 and the electronics housing 22 collectively contain an optical assembly 30 and a light source 32 (Fig. 2). By way of example, the optics housing 20 contains the optical assembly 30 and the light source 32, while the electronics housing 22 encloses supporting electrical connections for powering the light source 32. However, embodiments of the invention are not limited to specific locations of the optical assembly 30 and the light source 32. For example, embodiments of the invention contemplate the light source 32 being contained in the electronics housing 22.

With reference to the exemplary embodiment shown in Figs. 1 and 2, the optics housing 20 is a generally tubular body 34 that defines an end 36 from which light exits internally from the light source 32 along an optical axis 38. At an opposing end 40, the optics housing 20 is coupled to the electronics housing 22 such as via a threaded joint 42. The electronics housing 22 closes off the end 40 of the optics housing 20 to contain the optical assembly 30 and the light source 32 within the optics housing 20. Though capping the end 40, the electronics housing 22 provides an electrical connection point to a power source (not shown), such as by opening 44. The electronics housing 22 may also provide a connection point to the yoke 14. In that regard, by way of example only, as shown in Figs. 1 and 2, the electronics housing 22 includes an eyelet 46 that receives a fastener 48, such as a pin or a screw, by which the headlight 12 is pivotally attached to the yoke 14. The yoke 14 may, in turn, be pivotally attached to the TTL clip 16, such as by a screw 50, lock washer 52, and hex nut 54.

With continued reference to Fig. 2, the optics housing 20 contains the optical assembly 30 which, in the exemplary embodiment, includes multiple lenses 60, 62, 64. As shown, each optical axis of each of first singlet lens 60, second singlet lens 62, and third singlet lens 64 is aligned on the optical axis 38. In one embodiment, the optical axis 38 passes from the light source 32 through a center of curvature of each lens surface. When energized, light from the light source 32 is projected through each lens 60, 62, and 64 and out of the headlight 12 along the optical axis 38. In the exemplary embodiment, the optical axis 38 coincides with a longitudinal axis 66 of the optics housing 20. In other words, in the exemplary embodiment, the optical assembly 30 does not include a mirror or a prism by which the light is reflected or refracted before exiting the headlight 12. Advantageously, the light beam provides a uniform surface illumination.

More specifically, as shown in Figs. 1 and 2, the tubular body 34 has a conical configuration in which an outer dimension of the tubular body 34 enlarges from the end 40 toward the end 36. The tubular body 34 is configured to receive and arrange the lenses 60, 62, and 64 relative to the light source 32 at specific predetermined distances relative to one another to thereby provide an improved light beam. In that regard, and in the exemplary embodiment, the tubular body 34 defines an objective lens chamber 70 and a secondary lens chamber 72. As shown, the objective lens chamber 70 houses the objective lens 60, and the secondary lens chamber 72 houses each of the middle lens 62 and the light source lens 64. In one embodiment, the optical assembly 30 consists of the three lenses, that is, the objective lens, the middle lens 62, and the light source lens 64. In this embodiment, no other lenses are utilized, for example, the LED does not include an integral lens.

In the objective lens chamber 70, at the distal end 36 of the tubular body 34, a distal stop 74 defines a predetermined distance for mounting the lens 60 to the secondary lens chamber 72. As shown, the distal stop 74 may appear as a tab, lip, or other projection of the tubular body 34. The stop 74 is spaced from the end 36 and extends inwardly toward the longitudinal axis 66 to a distance sufficient to receive the lens 60 within the objective lens chamber 70 at a fixed position. The distal stop 74 positions the lens 60 at a specific distance from the lenses 62 and 64 and the light source 32. In the exemplary embodiment, the objective lens 60 is a singlet lens. The objective lens 60 is bi-convex having a radius of curvature in a range of 11 mm to 12 mm (left side in Fig. 2) and a radius of curvature in a range of 88 mm to 89 mm (right side in Fig. 2), a diameter in a range of 12 mm to 14 mm, and a thickness (T1) in a range of 3 mm to 4 mm . By way of comparison to singlet lenses, none of the lens 60, 62, 64 is a doublet lens in which two singlet lenses are paired together, such as being cemented together. Although doublet lenses may be air-spaced in which the two singlets are placed surface of one singlet in contact with the surface of the adjacent singlet or oiled together in which a thin coat of oil is between two singlets.

More specifically, the distal stop 74 is a predetermined distance along the optical axis 38 from a midpoint stop 76. As shown, the midpoint stop 76 is positioned at a junction 80 between the objective lens chamber 70 and the secondary lens chamber 72. As shown, the junction 80 may appear as a discontinuity in the thickness of the tubular body 34 in which an inside dimension of the objective lens chamber 70 abruptly reduces to an inside dimension of the secondary lens chamber 72. As shown, the middle lens 62 is received against the midpoint stop 76. By way of example, the predetermined distance from lens contact point on the distal stop 74 to lens contact point on midpoint stop 76 is in a range of 16 mm to 18 mm. By way of another example, the predetermined distance is 17.16 mm. The distal stop 74 and the midpoint stop 76 define a distance D1 between the surface of lens 60 to the nearest surface of lens 62. By way of example, D1 may range from 15 mm to 18 mm. In the exemplary embodiment, the distance D1 is 16.75 mm and the middle lens 62 is a singlet lens. By way of examples only and not limitation, the middle lens 62 is bi-convex having a radius of curvature in a range of 9.5 mm to 10 mm (left side in Fig. 2) and a radius of curvature in a range of 5.5 mm to 6.5 mm (right side in Fig. 2), a diameter in a range of 4.5 mm to 5.5 mm, and a thickness (T2) in a range of 1.5 mm to 2.5 mm.

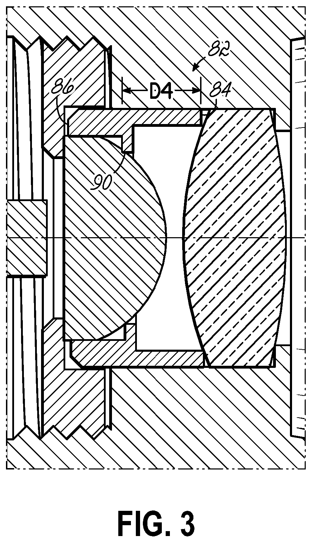

With reference to Figs. 2 and 3, in the exemplary embodiment, the light source lens 64 is spaced apart from lens 62 by a distance defined by a centering ring 82. The centering ring 82 may be slidably received in the secondary lens chamber 72. As shown in Fig. 3, the centering ring 82 is a tubular member having opposing ends 84, 86. Between the ends 84, 86, the centering ring 82 carries a light source stop 90. Similar to the distal stop 74 and midpoint stop 76, the light source stop 90 is a lip, tab, or inwardly extending projection. When inserted into the secondary lens chamber 72, the centering ring 82 abuts the lens 62 at end 84. The light source lens 64 is then received in the centering ring 82 and abuts the stop 90. In this way, the centering ring 82 spaces the light source lens 64 apart from the middle lens 62 by a distance D2 between the nearest surfaces of each of lens 62 and lens 64. While the centering ring 82 is shown as a one-piece structure (e.g., made of aluminum), embodiments of the invention are not limited to a single piece as multiple components are contemplated by which the light source lens 64 is spaced apart from the middle lens 62. By way of example, D2 may be less than 0.5 mm but greater than 0.05 mm and as a further example, D2 may range from 0.2 mm to 0.4 mm. In the exemplary embodiment, the distance D2 is 0.33 mm and the light source lens 64 is a singlet lens. By way of examples only and not limitation, the light source lens 64 is a plano-convex lens having a radius of curvature in a range of 1.5 mm to 2.5(left side in Fig. 2) and is flat on one side (right side in Fig. 2), a diameter in a range of 3.5 mm to 4.5 mm, and a thickness (T3) ina range of 1.5 mm to 2.5 mm. By way of example only, a distance D4 (Fig. 3) between the end 84 and the stop 90 of the centering ring 82 is in a range from 1 mm to 2.5 mm. By way of additional example, D4 is 1.47 mm.

With reference to Fig. 2, a retaining ring 92 captures the lens 64 against the centering ring 82. As shown, the retaining ring 92 may be received in the tubular body 34 proximate the end 40 via cooperating threads. The retaining ring 92 is spaced apart and so may not contact the end 86 of the centering ring 82. This arrangement ensures that each of the lens 62 and lens 64 and each of the lens 62 and lens 60 are maintained at the distance D2 and D1, respectively, from one another. The retaining ring 92 thereby also ensures that the distances D1 and D2 in the optical assembly 30 are fixed at their predetermined distances.

With continued reference to Fig. 2, in the exemplary embodiment the light source 32 is a light emitting diode (LED). As shown, the LED is mounted on a printed circuit board (PCB) 96. In the exemplary embodiment, the LED lacks an integral lens. Light emitted from the LED first impinges on the light source lens 64. Stated another way, light emitted from the LED is not first focused by a lens secured to the LED before impinging on the light source lens 64. The LED may have a die size of between 0.3 mm² and 2 mm². As shown, the PCB 96 may abut the end 40 of the tubular body 34 and so be housed within the electronics housing 22 with the light source 32 at least partly protruding into the optics housing 20. The PCB 96 is held in that abutting position by the electronics housing 22 when the electronics housing 22 is threaded onto the tubular body 34 at threaded joint 42. In the exemplary embodiment, the abutting position of the PCB 96 against end 40 of the optics housing 20 precisely sets the distance between the light source 32 and each of the first singlet lens 60, second singlet lens 62, and the third singlet lens 64. With this arrangement, the light source 32 is aligned on the optical axis 38 and is accurately spaced apart from the lens 64 by a predetermined distance D3. By way of example only, the distance D3 is in a range of 0.2 mm to 0.4 mm. By way of additional example, and not limitation, D3 is 0.33 mm. In the exemplary embodiment, the space between the lens 64 and the light source 32 lacks any other optical element, such as a lens built onto the LED. The spacing of the light source 32 relative to the lens 64 may be determined, for example, by a distance between the end 40 and the stop 76. By way of example and not limitation, the distance is in the range of 4 mm to 6 mm, and by way of additional example, the distance is 5.0 mm. In the exemplary embodiment, the LED has a die size of 1.1 mm by 1.1 mm, a mechanical size of 1.4 mm by 1.4 mm, and is available from Osram under the mark OSLON^{®} Pure 1414 with identifier GW VJLPL1 and color temperature 6500K.

The optical assembly 30, particularly the arrangement of the lenses 60, 62, and 64 relative to the LED 32 may produce a substantially uniform light beam and a substantially round, homogenous light spot at the target. By way of example only, and not limitation, characteristics of the light source 32 include luminous flux in the range from 40 lumens to 130 lumens, color temperature (K) in a range of 6300 to 6700 and by way of additional example in a range of 6360 to 6510, and color rendering index having a minimum of 90 and a typical (nominal) value of 91. As a further example and not limitation, electrical power for the LED 32 may range from 100 mA to 700 mA. As an additional example, and not limitation, electrical power for the LED 32 may range from 140 mA to 625 mA. And, as yet another example and not limitation, electrical power for the LED 32 may range from 145 mA to 555 mA.

While the present invention has been illustrated by a description of various preferred embodiments and while these embodiments have been described in some detail, it is not the intention of the inventor to restrict or in any way limit the scope of the appended claims to such detail. Additional advantages and modifications will readily appear to those skilled in the art. The various features of the invention may be used alone or in any combination depending on the needs and preferences of the user.

What is claimed is:

## Claims

1. A headlight of a type worn by medical and dental professionals, the headlight comprising:
a housing defining a first end opposite a second end and a passage extending between the first end and the second end;
a light source proximate the first end; and
an optical assembly at least partly in the passage of the housing, the optical assembly comprising:
a first singlet lens proximate the second end and defining an optical axis;
a second singlet lens disposed between the light source and the first singlet lens; and
a third singlet lens disposed between the light source and the second singlet lens,
wherein each of the second singlet lens and the third singlet lens are centered on the optical axis and the second singlet lens and the third singlet lens are spaced apart by a separation distance.

2. The headlight of claim 1, wherein the third singlet lens is a plano-convex lens and is oriented with a flat optical surface facing the light source.

3. The headlight of claim 1 or claim 2, wherein the first singlet lens and the second singlet lens are each bi-convex lenses.

4. The headlight of any preceding claim, wherein the optical assembly consists of:
the first singlet lens, the second singlet lens, and the third singlet lens.

5. The headlight of any preceding claim, further comprising:
a centering ring in the passage proximate the first end and in contact with the third singlet lens and positioning the third singlet lens on the optical axis.

6. The headlight of claim 5, wherein the centering ring includes a first stop and a second stop, the first stop contacting the second singlet lens and the second stop contacting the third singlet lens, the separation distance being defined by a first distance from the first stop to the second stop.

7. The headlight of claim 6, further comprising:
a retaining ring in the passage proximate the first end and in contact with the third singlet lens, the retaining ring capturing the third singlet lens against the second stop.

8. The headlight of any one of claims 5-7, further comprising:
a printed circuit board on which the light source is mounted,
wherein the printed circuit board is at the first end with the light source in the passage and the light source is spaced apart from the third singlet lens by a second distance.

9. The headlight of any one of claims 5-8, wherein the housing includes a third stop in the passage, and
wherein the first stop of the centering ring holds the second singlet lens against the third stop.

10. The headlight of claim 9, wherein a third distance from the third stop to the first end is in a range of 4 mm to 6 mm.

11. The headlight of claim 9 or claim 10, wherein the housing includes a fourth stop proximate the second end, the first singlet lens being in contact with the fourth stop.

12. The headlight of any one of claims 9-11, wherein a fourth distance from the third stop to the fourth stop is in a range of 16 mm to 18 mm.

13. The headlight of any preceding claim, wherein the light source produces a luminous flux of from 40 lumens to 130 lumens.

14. The headlight of any preceding claim, wherein the light source produces a color temperature of from 6300 K to 6700 K.

15. The headlight of any preceding claim, wherein the housing defines a longitudinal axis and the optical axis coincides with the longitudinal axis.
